# EUROPEAN PATENT SPECIFICATION

(11) **EP 2 708 228 B1**
(45) Date of publication and mention of the grant of the patent: **16.05.2018**
(21) Application number: 12183989.8
(22) Date of filing: 12.09.2012
(51) Int. Cl.: A61K 31/02, A61P 27/02

(54) **Eye wash compositions**
Augenspülungszusammensetzungen
Compositions de lavage oculaire

(43) Date of publication of application: 19.03.2014
(73) Proprietor: Novaliq GmbH, 69120 Heidelberg (DE)
(72) Inventor: Günther, Bernhard, 69221 Dossenheim (DE); Theisinger, Bastian, 68239 Mannheim (DE); Theisinger, Sonja, 68239 Mannheim (DE); Scherer, Dieter, 4242 Laufen (CH)
(74) Representative: Pharma Concepts GmbH

(56) References cited:
- EP-A1- 2 332 525
- EP-A1- 2 347 749
- EP-A1- 2 462 921
- WO-A1-2011/073134
- WO-A2-2011/113855
- KLAER-DISSARS ULRIKE ET AL: "Partially fluorinated alkanes as wash out solutions after removal of silicone oil used as an ocular endotamponade", GRAEFE'S ARCHIVE FOR CLINICAL AND EXPERIMENTAL OPHTHALMOLOGY, vol. 242, no. 3, March 2004 (2004-03), pages 218-222, XP002685984, ISSN: 0721-832X
- MEINERT H ET AL: "Semifluorinated alkanes--a new class of compounds with outstanding properties for use in ophthalmology", EUROPEAN JOURNAL OF OPHTHALMOLOGY, MILAN, IT, vol. 10, no. 3, 1 July 2000 (2000-07-01), pages 189-197, XP009134460, ISSN: 1120-6721

## Description

### BACKGROUND

Contamination of the eye by foreign agents such as physical or chemical irritants can cause extreme discomfort, irritation and pain. The ocular response is usually pain and excess reflex lacrimation and can further lead to blepharospasm, periorbital oedema as well as conjunctival erythema. However, further injury to the ocular surface may also ensue, caused for example by excessive rubbing of the irritated eye. In the case of chemical agents, the extent of injury is dependent on the concentration, type, length and manner of chemical agent exposure. Ocular injuries may include corneal oedema to more severe conditions with long term effects such as corneal ulceration and scarring, opacification and vascularization.

The circumstances in which the eye can become contaminated can vary, from incidental or accidental occurrences in the workplace or home environment, to more deliberate situations such as the spreading of chemical agents during warfare or for enforcement (e.g. riot or crowd control) purposes. As such, the range of chemical agents that the eye may encounter is also diverse, and includes agents derived from household or industrial materials, corrosive acids or alkalines, vesicants, irritants and lachrymators. Lachrymators, commonly referred to as 'tear gases', are often deployed for anti-personnel or crowd control purposes and include compounds such as capsaicin and capsaicinoids, o-chlorobenzylidene malononitrile (CS), chloroacetophenone (CN) or dibenzooxazepine (CR). Foreign particles, i.e. solid particulates such as those derived from pollutants, dust, soot, pollen and any form of fine-grained or powdered materials are also sources of irritation and may become adhered or embedded in the eye mucosa and surrounding ophthalmic tissue. A chemical agent can be in solid particulate form and may have both a physical and physiological irritating effect on the eye.

In general, the first line of treatment following contact of the eye with a chemical agent or particles is immediate irrigation with fluids, to dilute and/or remove and wash out the contaminant. Commonly used fluids are water or saline. Ideally, the aqueous fluids that are to be administered to an often inflamed (or already injured) eye should be sterile. Unfortunately aqueous solutions are often prone in supporting microbial growth. As such, in order to prolong storage, aqueous solutions which are used for eye wash purposes are often preserved with preservatives e.g. benzalkonium chloride, which are also irritants, or require specific types of storage containers (e.g. individual dose units) or dispensing means, which are not cost-effective, to prevent contamination during use.

While for water-soluble substances, the instant diluting effect from administering aqueous based solutions may have an immediate impact in preventing further irritation, for substances that are non-soluble or poorly soluble in water and hydrophobic, significant volumes of such solutions as well as lengthy irrigation times are often required for adequate relief. Prolonged irrigation with water may lead to temporary corneal oedema. Also, in emergency situations or in certain environments, the availability of large quantities of required irrigating fluid may be limited.

Other fluids or additives in addition or in combination to aqueous solutions may be used to remove more hydrophobic chemical agents and particles from the eye, but with limitations. For example, the use of common household remedies such as soaps or shampoo and other surfactants to aid in the removal of hydrophobic substances from the eye is limited by the inherent irritating nature of those items on the eye mucosa. Emulsions and the like containing an oily phase are also disadvantaged, because the oily components with different refractive index from water can lead to blurriness and poor vision after use. The use of classical non-aqueous organic solvents (for example ethanol) is also usually not contemplated due to their irritant properties and/or inherent toxicities.

A common treatment approach following initial fluid irrigation with aqueous solutions is physical examination of the eye, for any particulate material that may remain unperturbed by the wash-out fluid. This is usually performed by a medical practitioner through light or slit-lamp examination. Solid particles can remain embedded or hidden within the ophthalmic tissue in the cases where the aqueous solution has failed to properly reach or wet and dissolve the contaminant. Certain types of solid particulates, for example soot, repel water and are particularly difficult to wet. The use of a fan to blow dry air into the eyes is often suggested, for example for those contaminated with the riot control agent CS, a crystalline powder that is often dispersed as an aerosol or a spray. This is however not practical and in the case of a chemical agent such as CS, may lead to undesired further dispersing of the agent.

It is therefore an object of the present invention to provide a novel composition which is useful as a vehicle for the removal of chemical agents and foreign particles from the eye and which at the same time addresses and overcomes the various issues and at least one of the limitations or disadvantages associated with prior art methods and formulations.

US 6,262,126 discloses the use of semifluorinated alkanes as suitable diluent and intraocular wash fluid for silicone oil which has been used as a retinal tamponade.

WO 2011/073134 describes ophthalmic compositions comprising semifluorinated alkanes for topically delivering immunosuppressant macrolides to the eye. However, it does not teach an effect of semifluorinated alkanes on foreign agents contaminating an eye, in particular solid particles and agents which may be detrimentally chemically active against the ocular surface.

It is an object of the invention to provide an improved composition which is useful for the removal of chemical agents and particles from the eye, in particular agents and particles which are hydrophobic, insoluble and/or poorly soluble in water. In a further aspect, it is an object of the invention to provide a kit comprising a composition for removal of chemical agents and particles from the eye which overcomes one or more of the disadvantages of prior art. Further objects of the invention will become clear on the basis of the following description, examples, and patent claims.

### SUMMARY OF THE INVENTION

The invention provides a liquid composition comprising a semifluorinated alkane for use as a medicine for the removal of a foreign agent from the surface of an eye or from the surface of the cornea, the tear fluid, the lacrimal sac, conjunctiva of the sclera and eyelids, wherein the semifluorinated alkane is selected from F4H5, F4H6, F6H4, F6H6, F6H8 and F6H10.

The foreign agent may be solid, gaseous or liquid, and may comprise a physical or chemical irritant, such as a corrosive, a vesicant, or a lachrymatory agent. The foreign agent or the irritant may be hydrophobic or poorly water-soluble, and thus difficult to remove using conventional means.

In a further aspect, the invention provides a composition for use in the removal of a foreign agent from the surface of an eye or from the surface of the cornea, the tear fluid, the lacrimal sac, conjunctiva of the sclera and eyelids. The use includes a step of applying a composition comprising a semifluorinated alkane selected from F4H5, F4H6, F6H4, F6H6, F6H8 and F6H10, to the surface of the eye or surface of the cornea, the tear fluid, the lacrimal sac, conjunctiva of the sclera and eyelids such as to wash out the foreign agent. This may be combined with a further step in which an aqueous eye wash composition is used to rinse the eye or ophthalmic tissue, and/or it may be combined with a step of mechanically removing the foreign agent, or some of it.

### DETAILED DESCRIPTION OF THE INVENTION

In a first aspect, the invention provides a liquid composition comprising a semifluorinated alkane selected from F4H5, F4H6, F6H4, F6H6, F6H8 and F6H10 for use as a medicine for the removal of a foreign agent from the surface of an eye or from the surface of the cornea, the tear fluid, the lacrimal sac, conjunctiva of the sclera and eyelids.

Semifluorinated alkanes are linear or branched alkanes some of whose hydrogen atoms have been replaced by fluorine. The semifluorinated alkanes (SFAs) used in the present invention are composed of at least one non-fluorinated hydrocarbon segment and at least one perfluorinated hydrocarbon segment. Particularly useful are SFAs which have one non-fluorinated hydrocarbon segment attached to one perfluorinated hydrocarbon segment, according to the general formula F(CF₂]ₙ(CH₂)ₘH. SFAs which are useful in the context of the present invention and their preparation are also described in EP-A 965 334, EP-A 965 329 and EP-A 2 110 126, the disclosure of which documents is incorporated herein.

Another nomenclature which is used herein refers to the above-mentioned SFAs having two or three segments as RFRH and RFRHRF, respectively, wherein R_{F} designates a perfluorated hydrocarbon segment, R_{H} designates a non-fluorinated segment. Alternatively, the compounds may be referred to as FnHm and FnHmFo, respectively, wherein F means a perfluorated hydrocarbon segment, H means a non-fluorinated segment, and n, m and o is the number of carbon atoms of the respective segment. For example, F3H3 is used for perfluoropropylpropane. Moreover, this type of nomenclature is usually used for compounds having linear segments. Therefore, unless otherwise indicated, it should be assumed that F3H3 means 1-perfluoropropylpropane, rather than 2-perfluoropropylpropane, 1-perfluoroisopropylpropane or 2-perfluoroisopropylpropane.

The unexpected advantages of the SFA in the context of the use according to the present invention are believed to relate to their unique properties; for example, their excellent physiological tolerability when administered topically, even to sensitive mucosae such as the mucosae of the eye, but also their ability to wet hydrophobic particles such as soot, their solubilising capacity for various poorly water-soluble chemical irritants and the like, allowing their rapid removal from the eye. Moreover, they do not lead to blurred vision like most non-aqueous liquids (such as oils) do, as their refractive index is close to that of physiological tear fluid. Also, semifluorinated alkanes do not support microbial growth. Thus they may be used for ophthalmic administration without preservatives, which are known to act as irritants to the eye and which should be avoided in particular for an eye which is already irritated.

The semifluorinated alkane in the composition to be used according to the invention are selected from F(CF₂)₄(CH₂)₅H (abbreviated F4H5), F(CF₂)₄(CH₂)₆H (abbreviated F4H6), F(CF₂)₆(CH₂)₄H (abbreviated F6H4), F(CF₂)₆(CH₂)₆H (abbreviated F6H6), F(CF₂)₆(CH₂)₈H (abbreviated F6H8) and F(CF₂)₆(CH₂)₁₀H (abbreviated F6H10).

In a simple but nevertheless advantageous embodiment, the liquid composition to be used according to the invention substantially consists of an SFA as described above. Alternatively, the composition may comprise one or more further constituents. For example, it may comprise a further semifluorinated alkane which is different from the first semifluorinated alkane, but which may be selected according to the same preferences as described above. Optionally, the further semifluorinated alkane may also be a solid compound at normal conditions. If so, it is preferred that it is soluble in the first semifluorinated alkane, and that the composition is a clear solution.

The incorporation of two semifluorinated alkanes in the composition may bring about several further advantages. For example, combining semifluorinated alkanes may lead to a combination of excellent wetting and solubilisation capacity with further optimised optical properties at a viscosity which is appropriate for the intended use as an eye wash composition.

Preferably, the liquid semifluorinated alkane or mixture of semifluorinated alkanes is the major constituent of the composition. If a further component is present, such component is preferably dissolved in the semifluorinated alkane or mixture of semifluorinated alkanes. As used herein, the major component is the component that represents the largest fraction of all components in the composition. According to further preferred embodiments, the liquid semifluorinated alkane or mixture of semifluorinated alkanes represents at least 90 wt.-% of the composition, or at least 95 wt.-%, or at least 99 wt.-%. As mentioned above, it is also preferred to use a composition comprising only a liquid semifluorinated alkane or mixture of semifluorinated alkanes, without comprising any further components.

Optionally, the composition may comprise a further component selected from an anti-inflammatory or local anaesthetic agent. Examples of anti-inflammatory agents include diclofenac, flurbiprofen or bromfenac. Examples of local anaesthetic agents include tetracaine, proparacaine, lidocaine, or oxybuprocaine. Preferably, the further component selected from an anti-inflammatory or local anaesthetic is dissolved in the semifluorinated alkane or mixture of semifluorinated alkanes.

The composition may be used to remove any type of foreign agent from an eye or ophthalmic tissue which is removable by externally applied means. Thus, any foreign agent that has contaminated a part of an eye which is accessible for an eye wash, for example an agent that has contaminated the surface of the cornea, the tear fluid, the lacrimal sac, and in particular the conjunctiva of the sclera and the eyelids, may be removed using the composition.

The foreign agent, or contaminant, may be a solid, gaseous or liquid agent. Common solid contaminants include, for example, small particles such as sand, dust, in particular fine dust, such as carbon dust, soot, saw dust, stone dust, flour, and swarf; larger particles such as wood and metal chips or shavings, particulate solid chemicals and the like. Gaseous foreign agents or contaminants include, for example, volatile organic compounds, aerosols, household sprays, technical sprays, chemical or laboratory gases such as hydrochloric acid, sulphur dioxide, irritant gases used by police or armed forces e.g. as riot control agents, vesicants, and lachrymatory agents, such as tear gas (2-chlorobenzalmalononitrile, CS), chloroacetophenone (CN), chloropicrin (PS), bromobenzenecyanide (CA), dibenz-(b,f)-1,4-oxazepine (CR), or oleoresin capsicum or capsaicin (pepper spray, OC), some of which are used as aerosolised liquid solutions which may further comprise one or more solvent or propellant. Liquid foreign agents or contaminants include, for example, hot or cold kitchen or household liquids, such as oils, cleaning agents, solvents, polishes, decalcifying agents, lubricants, waterproofing agents, dyes, fertilisers; chemicals or laboratory liquids such as liquid acids, bases, organic solvents, corrosives, and the like. Typically, the foreign agent or contaminant acts as a mechanical or chemical irritant on the eye or ophthalmic tissue.

The composition may be advantageously used, in particular, for the removal of foreign agents which are not easily removable by conventional aqueous eye wash solutions due to their hydrophobicity, or poor water solubility. In fact, some common contaminants such as capsaicin or soot are highly hydrophobic and normally very difficult to even wet and disperse in a conventional eye wash solution. In contrast, such agents are much more readily wetted, dispersed and washed out from the eye by the composition of the invention.

The use of the composition according to the invention typically involves its topical administration to the affected eye or ophthalmic tissue. Depending on the amount and nature of the contaminant, the composition may be instilled or placed on the surface of the eye or ophthalmic tissue in a dropwise manner, or the eye or tissue may be continuously irrigated with the composition for a period of time sufficient to remove a substantial fraction of the contaminant and/or to achieve subjective relief or soothing.

Optionally, the composition may be used in combination with a further means for removing the foreign agent or contaminant. For example, a conventional aqueous eye wash solution may be used before or after the composition comprising the semifluorinated alkane, and/or a step of mechanically removing a foreign agent may be added. Combination use may thus be particularly advantageous in the case where an eye is affected by more than a single contaminant, or if a contaminant contains more than one irritating components. For example, the composition comprising the semifluorinated alkane may be used to remove or wash out a hydrophobic mechanical or chemical irritant before or after an aqueous eye wash solution is administered to remove a hydrophilic contaminant or component of the contaminant, or to fully neutralise the conjunctiva after a substantial pH shift caused by the contaminant. It may also be useful to first remove larger mechanical agents such as metal or wooden chips or shavings by mechanical means and subsequently using the composition comprising the semifluorinated alkane in order to remove or wash out the finer particles, e.g. the dust fraction of the contaminant.
The composition is preferably provided in sterile form in a container which allows easy administration to the eye, or as a kit comprising such container and the composition. The container may be optionally equipped with a dispensing means adapted for topical administration of the composition to an eye or ophthalmic tissue. Optionally, the kit may also include an application means which is not part of the container, such as an eyecup, eyebath, or similar vessel to fit the eye. Preferably, such application means, or application aid, is also provided in sterile form within the kit.

## Claims

1. A liquid composition comprising a semifluorinated alkane selected from F4H5, F4H6, F6H4, F6H6, F6H8 and F6H10, for use as a medicine for the removal of a foreign agent from the surface of an eye or from the surface of the cornea, the tear fluid, the lacrimal sac, conjunctiva of the sclera and eyelids.

2. The composition for use of claim 1, wherein the foreign agent is selected from solid, gaseous and liquid agents.

3. The composition for use of any preceding claim, wherein the foreign agent comprises a physical or chemical irritant.

4. The composition for use of claim 3, wherein the foreign agent and/or the physical or chemical irritant is hydrophobic, insoluble or poorly soluble in water.

5. The composition for use of claim 3 or 4, wherein the physical or chemical irritant is selected from corrosives, vesicants, and lachrymatory agents.

6. The composition for use of any preceding claim, wherein the use comprises the topical administration of the composition to the eye.

7. The composition for use as defined in claim 1, wherein the use comprises a step of applying the composition to the surface of the eye such as to wash out the foreign agent from the eye.

8. The composition for use of claim 7, wherein the use further comprises a step of rinsing the eye with an aqueous composition and/or of removing the foreign agent with mechanical means.

9. A kit comprising a composition for use as defined in claim 1 and a container for holding the composition, wherein the container has a dispensing means adapted for topical administration of the composition to an eye.

## Patentansprüche

1. Eine flüssige Zusammensetzung umfassend ein semifluoriertes Alkan ausgewählt aus F4H5, F4H6, F6H4, F6H6, F6H8 und F6H10, zur Verwendung als Arzneimittel für die Entfernung eines Fremdstoffs von der Augenoberfläche oder von der Hornhautoberfläche, der Tränenflüssigkeit, dem Tränensack, der Bindehaut der Sklera und Augenlider.

2. Die Zusammensetzung zur Verwendung nach Anspruch 1, wobei der Fremdstoff ausgewählt ist aus festen, gasförmigen und flüssigen Stoffen.

3. Die Zusammensetzung zur Verwendung nach einem der vorhergehenden Ansprüche, wobei der Fremdstoff ein physikalisches oder chemisches Irritans umfasst.

4. Die Zusammensetzung zur Verwendung nach Anspruch 3, wobei der Fremdstoff und/oder das physikalische oder chemische Irritans hydrophob, unlöslich oder schwer löslich in Wasser ist.

5. Die Zusammensetzung zur Verwendung nach Anspruch 3 oder 4, wobei das physikalische oder chemische Irritans ausgewählt ist aus ätzenden, blasenziehenden und tränenreizenden Stoffen.

6. Die Zusammensetzung zur Verwendung nach einem der vorhergehenden Ansprüche, wobei die Verwendung die topische Verabreichung der Zusammensetzung auf das Auge umfasst.

7. Die Zusammensetzung zur Verwendung gemäß Anspruch 1, wobei die Verwendung einen Schritt umfasst, bei dem die Zusammensetzung auf die Oberfläche des Auges derart aufgetragen wird, dass der Fremdstoff aus dem Auge ausgewaschen wird.

8. Die Zusammensetzung zur Verwendung nach Anspruch 7, wobei die Verwendung weiterhin einen Schritt umfasst, bei dem das Auge mit einer wässrigen Zusammensetzung gespült wird und/oder der Fremdstoff mit einem mechanischen Mittel entfernt wird.

9. Ein Kit, umfassend eine Zusammensetzung zur Verwendung gemäß Anspruch 1 und ein Behälter zum Aufnahme der Zusammensetzung, wobei der Behälter eine Abgabevorrichtung hat, die an eine topische Verabreichung der Zusammensetzung auf ein Auge angepasst ist.

## Revendications

1. Composition liquide comprenant un alcane semi-fluoré choisi parmi F4H5, F4H6, F6H4, F6H6, F6H8 et F6H10, ladite composition étant destinée à être utilisée comme médicament pour éliminer un agent étranger de la surface d'un oeil ou de la surface de la cornée, le liquide lacrymal, le sac lacrymal, la conjonctive de la sclérotique et des paupières.

2. Composition destinée à être utilisée selon la revendication 1, l'agent étranger étant choisi parmi les agents solides, gazeux et liquides.

3. Composition selon l'une quelconque des revendications précédentes, l'agent étranger comprenant un irritant physique ou chimique.

4. Composition selon la revendication 3, l'agent étranger et/ou l'irritant physique ou chimique étant hydrophobe, insoluble ou peu soluble dans l'eau.

5. Composition destinée à être utilisée selon la revendication 3 ou 4, l'irritant physique ou chimique étant choisi parmi les agents corrosifs, les vésicants et les agents lacrymogènes.

6. Composition selon l'une quelconque des revendications précédentes, l'utilisation comprenant l'administration topique de la composition à l'oeil.

7. Composition destinée à être utilisée selon la revendication 1, l'utilisation comprenant une étape d'application de la composition à la surface de l'oeil de manière à éliminer l'agent étranger de l'oeil par lavage.

8. Composition selon la revendication 7, l'utilisation comprenant en outre une étape de rinçage de l'oeil avec une composition aqueuse et/ou d'élimination de l'agent étranger avec des moyens mécaniques.

9. Kit comprenant une composition destinée à être utilisée de la manière définie dans la revendication 1 et conteneur destiné à conserver la composition, le conteneur comportant un moyen de distribution adapté à l'administration topique de la composition à un oeil.
